(19) Europäisches Patentamt
European Patent Office
Office européen des brevets

(11) EP 1 069 140 A1

(12) **EUROPEAN PATENT APPLICATION**
published in accordance with Art. 158(3) EPC

(43) Date of publication:
**17.01.2001 Bulletin 2001/03**

(21) Application number: **97909588.2**

(22) Date of filing: **22.10.1997**

(51) Int. Cl.$^7$: **C08F 20/34**, C08F 20/60, C08F 8/12, C07C 251/08, C07C 219/08, C07C 249/02, C07C 213/02, C02F 11/14, B01D 21/01

(86) International application number:
**PCT/JP97/03824**

(87) International publication number:
**WO 99/20667 (29.04.1999 Gazette 1999/17)**

(84) Designated Contracting States:
**FR**

(71) Applicant:
**SANYO CHEMICAL INDUSTRIES, LTD.**
**Kyoto-shi, Kyoto 605 (JP)**

(72) Inventors:
- **NODA, Kimihiko,**
  **Sanyo Chemical Industries, Ltd.**
  **Kyoto-shi, Kyoto 605 (JP)**
- **SATO, Masaaki,**
  **Sanyo Chemical Industries, Ltd.**
  **Kyoto-shi, Kyoto 605 (JP)**
- **SATAKE, Munekazu,**
  **Sanyo Chemical Industries, Ltd.**
  **Kyoto-shi, Kyoto 605 (JP)**
- **KAWAGUCHI, Shinobu,**
  **Sanyo Chemical Industries, Ltd**
  **Kyoto-shi, Kyoto 605 (JP)**

(74) Representative:
**Nevant, Marc et al**
**Cabinet Beau de Loménie,**
**158, rue de l'Université**
**75340 Paris Cedex 07 (FR)**

# (54) WATER-SOLUBLE POLYMER, PROCESS FOR THE PRODUCTION OF THE SAME, AND USE THEREOF AS POLYMERIC COAGULANT

(57) The object of the present invention is to provide a novel high-performance polymer which can be used as a high molecular weight flocculant in centrifugation or beltpress dewatering, for instance, to give a sufficient degree of dehydration.

The present invention provides a water-soluble vinyl polymer (A) consisting essentially of constituent monomer units of a radical-polymerizable (meth)acrylic monomer (m),
containing a primary amine salt group,
said polymer (A) having colloid equivalent values a, b and c, at pH of 4, 7 and 10, respectively, providing the ratio of b/a in the range of 0.1 - 0.5 and the ratio of c/a in the range of 0 - 0.1.

EP 1 069 140 A1

## Description

TECHNICAL FIELD

The present invention relates to a water-soluble vinyl polymer comprising a primary amine salt group-containing radical-polymerizable (meth) acrylic monomer as an essential constituent monomer; a high-molecular weight flocculant comprising this polymer; and methods of producing this polymer, a starting monomer and intermediates.

More particularly, it relates to a novel water-soluble vinyl polymer comprising a primary amine salt group-containing radical-polymerizable (meth) acrylic monomer as an essential constituent monomer and useful as a high-molecular weight flocculant and further as a paper strength-improving agent, a paper drainage aid and an antistatic agent; a high-molecular weight flocculant comprising this polymer and allowing to attain a high degree of dehydration when used in dehydration, such as centrifugation or beltpress dewatering, of organic sludges resulting from microbiological treatment of excreta; a process for producing this polymer using a ketimine-containing (meth)acrylate as a precursor; and methods of producing the ketimine-containing (meth)acrylate, a primary amine salt group-containing (meth)acrylate and a polymer or copolymer comprising units of the ketimine-containing (meth) acrylate, which are precursors and intermediates of the above polymer.

The term "(meth)acrylate" as used herein means an acrylate and/or methacrylate, and similar expression are used hereinafter.

BACKGROUND ART

In dehydrating organic sludges resulting from microbiological treatment of sewage, excreta and the like, high-molecular cationic flocculants, such as polymethacryloyloxyethyltrimethylammonium chloride, acrylamide-acryloyloxyethyltrimethylammonium chloride copolymers and polyvinylamidines, have so far been used widely. Further, high-molecular amphoteric flocculants, such as acrylamide-acrylic acid-acryloyloxyethyltrimethylammonium chloride copolymers, have recently been proposed (Japanese Kokai Sho-63-260928).
In the prior art, however, a sufficient degree of dehydration has not been attained in centrifugation or beltpress dewatering, for instance. Particularly in recent years, degree of dehydration is apt to become worsened accompanied with significant increase of organic matter contents in sludges and advanced rot. The decreases in dehydration degree lead to increased water contents in cakes and, accordingly, there arises the problem of increased fuel cost of incineration of the cakes.

SUMMARY OF THE INVENTION

A primary object of the present invention is to provide a novel high-performance polymer, capable of being used as a high-molecular weight flocculant in dehydration, such as centrifugation or beltpress dewatering, to give a sufficient degree of dehydration.

Another object of the invention is to provide a high-molecular weight flocculant, comprising the above novel polymer, capable of giving a sufficient dehydration degree in centrifugation or beltpress dewatering, for instance.

Still another object of the invention is to provide industrially advantageous methods of producing the above novel polymer as well as precursors and intermediates therefor.

The present invention thus provides the following [1] to [6]:

[1] <u>Novel water-soluble vinyl polymer (A)</u>

A water-soluble vinyl polymer (A) consisting essentially of constituent monomer units of a radical-polymerizable (meth)acrylic monomer (m), containing a primary amine salt group, preferably a monomer (m1) represented by the general formula (1), and having colloid equivalent values a, b and c at pH of 4, 7 and 10, respectively, providing the ratio of b/a in the range of 0.1 - 0.5 and the ratio of c/a in the range of 0 - 0.1.

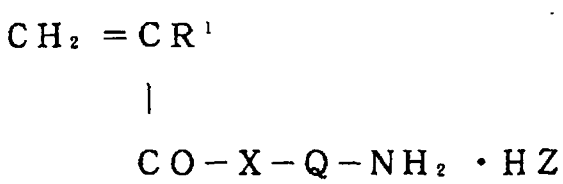

(1)

wherein $R^1$ is a hydrogen atom or a methyl group, X is an oxygen atom or an imino group, preferably an oxygen atom, Q is an alkylene or hydroxyalkylene group containing 2 to 4 carbon atoms, preferably an ethylene group, and Z is selected from the group consisting of Cl, Br, I, $NO_3$, $1/2SO_4$, $CH_3SO_3$, $H_2PO_4$ and $CH_3COO$.

[2] Use of said water-soluble vinyl polymer (A) as a high-molecular weight flocculant

Use of the water-soluble vinyl polymer (A) mentioned above under [1] as a high molecular weight flocculant, preferably as a sludge dehydrant.

[3] Novel process for producing said water-soluble vinyl polymer (A) using a ketimine-containing (meth)acrylate as a starting material

A process for producing said water-soluble vinyl polymer (A) mentioned above under [1]

comrising hydrolyzing and neutralizing, with an acidic aqueous solution, a polymer or copolymer derived from a ketimine-containing (meth)acrylate (n), or
hydrolyzing and neutralizing, with an acidic aqueous solution, the ketimine-containing (meth)acrylate (n) represented by the general formula (2):

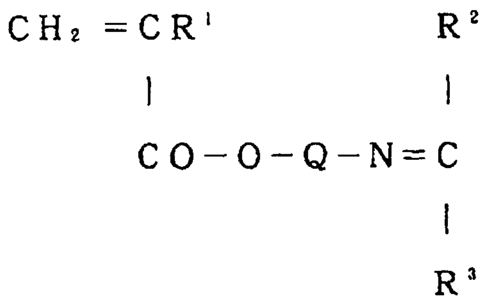

(2)

wherein $R^1$ is a hydrogen atom or a methyl group, Q is an alkylene or hydroxyalkylene group containing 2 to 4 carbon atoms, preferably an ethylene group, and $R^2$ and $R^3$ are alkyl groups containing 1 to 4 carbon atoms, preferably $R^2$ is an ethyl group and $R^3$ is a methyl group;
followed by polymerizing or copolymerizing the neutralization product.

[4] Process for producing said ketimine-containing (meth)acrylate as starting material

A process for producing the ketimine-containing (meth)acrylate (n) of the above general formula (2)

which process comprises carrying out transesterification, with an alkyl (meth)acrylate, of a primary amino alcohol-derived ketimine represented by the following general formula (4):

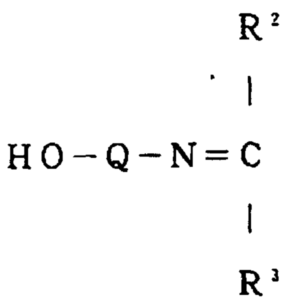

(4)

wherein Q is an alkylene or hydroxyalkylene group containing 2 to 4 carbon atoms, preferably an ethylene group, and $R^2$ and $R^3$ are alkyl groups containing 1 to 4 carbon atoms, preferably $R^2$ is an ethyl group and $R^3$ is a methyl group.

[5] Process for producing a primary amine salt group-containing (meth)acrylate intermediate

A process for producing a primary amine salt group-containing (meth)acrylate ester (m11) represented by the general formula (3):

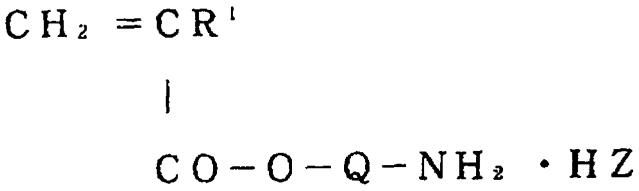

(3)

wherein $R^1$ is a hydrogen atom or a methyl group, Q is an alkylene or hydroxyalkylene group containing 2 to 4 carbon atoms, preferably an ethylene group, and Z is selected from the group consisting of Cl, Br, I, $NO_3$, $1/2SO_4$, $CH_3SO_3$, $H_2PO_4$ and $CH_3COO$;

which process comprises further hydrolyzing and neutralizing, with an acidic aqueous solution, the ketimine-containing (meth)acrylate (n) obtainable by the process for producing mentioned above under [4].

[6] Process for producing an intermediate polymer or copolymer comprising a ketimine-containing (meth)acrylate unit

A process for producing a polymer or copolymer comprising a ketimine-containing (meth)acrylate unit

which comprises polymerizing the ketimine-containing (meth)acrylate (n) obtainable by the process for producing mentioned above under [4] or
copolymerizing the ketimine-containing (meth)acrylate (n) with another vinyl monomer (k).

DETAILED DESCRIPTION OF THE INVENTION

In the following, the present invention is described in detail.
Preferred examples of the primary amine salt group-containing radical-polymerizable (meth) acrylic monomer (m) to be used in the practice of the present invention are monomers (m1) represented by the above general formula (1).
Among the monomers (m1), monomers (m11) containing X of an oxygen atom represented by the above general formula (3), are preferred. Among them, more preferred are ones containing Q of an ethylene group, because of ease of industrial production.
Illustrative specific examples of the above monomer (m1) include aminoethyl (meth)acrylate hydrochloride, aminoethyl (meth)acrylate sulfate, aminopropyl (meth)acrylamide hydrochloride and the like. One or more of these may be used. Among them, preferred are aminoethyl (meth)acrylate hydrochloride and aminoethyl (meth)acrylate sulfate, which have X of an oxygen atom and Q of an ethylene group. More preferred are aminoethyl (meth)acrylate hydrochloride, particularly aminoethyl methacrylate.
The above water-soluble vinyl polymer (A) may be a homopolymer of the monomer (m) or a copolymer thereof with another vinyl monomer (k) as used generally.
Examples of the other vinyl monomer (k) include nonionic vinyl monomers such as acrylamide, acrylonitrile and vinylpyrrolidone; anionic vinyl monomers such as acrylic acid (salt), acrylamidomethylpropanesulfonic acid (salt) and itaconic acid (salt); and cationic vinyl monomers such as (meth)acryloyloxyethyltrimethylammonium chloride, (meth)acryloyloxyethyldimethylbenzylammonium chloride, (meth)acryloylaminoethyltrimethylammonium chloride, dimethyldiallylammonium methyl sulfate and vinylpyridine. One or more of these may be used.
Among these, preferred are acrylamide and cationic vinyl monomers such as (meth)acryloyloxyethyltrimethylammonium chloride, particularly acrylamide and (meth)acryloyloxyethyltrimethylammonium chloride, from the industrial viewpoint.
The term "acrylic acid (salt)", for instance, as used herein means acrylic acid and/or an acrylic acid salt, and the same shall apply hereinafter.
In case said monomer (m) is copolymerized with said other vinyl monomer (k), the ratio of the monomer (m) in the copolymerization can be selected arbitrarily in answer to the intended purpose. When a cationic vinyl monomer is contained as said other vinyl monomer (k), the content of the monomer (m) is preferably not less than 50 mole %,

more preferably not less than 70 mole %, still more preferably not less than 80 mole %, based on the total amount of the cationic vinyl monomers, i.e. said monomer (m) and said cationic vinyl monomer, in the copolymer, from the viewpoint of performance characteristics of the polymer, in particular the dehydration degree when used as a high-molecular weight flocculant.

For the same reasons, the content of the monomer (m) based on the total of vinyl monomers including nonionic and anionic monomers, namely the total of said monomer (m) and said other vinyl monomer (k), is preferably not less than 50 mole %, more preferably not less than 70 mole %, particularly not less than 80 mole %.

Since the water-soluble vinyl polymer (A), in accordance with the present invention, contains the monomer (m) as the essential constituent monomer, the water-soluble vinyl polymer (A) has colloid equivalent values a, b and c at pH of 4, 7 and 10, respectively, such that the ratios b/a and c/a are 0.1 to 0.5 and 0 to 0.1, respectively.

The colloid equivalent value a at pH 4 of the above water-soluble vinyl polymer (A) is preferably not less than 5.0, more preferably not less than 5.5 to 13, especially 6.0 to 12, in view of dehydration performance when used as a high-molecular weight flocculant. By using the monomer (m) as the essential constituent monomer, it is possible to obtain easily polymers showing a colloid equivalent value a of not less than 5.0 at pH 4.

In the practice of the present invention, the colloid equivalent value can be determined by colloidal titration as shown below.

(1) Preparation of 50 ppm aqueous sample solution

0.2 gram (on the dry basis) of the sample is exactly weighed, placed in an erlenmeyer flask and dissolved into 100 ml of deionized water. Furthermore, 10 ml of this solution is taken and 390 ml of deionized water is added to make a homogeneous solution, which is a measurement sample.

(2) Determination of colloid equivalent value

A 100-ml portion of the measurement sample is transferred into a conical beaker, and a 0.5% aqueous solution of sodium hydroxide is gradually added with stirring to adjust the sample to the measurement pH. Then, 2 or 3 drops of toluidine blue is added as an indicator, followed by carrying out titration with N/400 polyvinyl sulfuric acid potassium (N/400 PVSK). The rate of dropping is 2 ml/min and the end point is the point at which the measurement sample changes its color from blue to purplish red, which color is maintained for not less than 30 seconds.

(3) Blank test

The same procedure as mentioned above under (2) is performed with 100 ml of deionized water.

(4) Calculation

The colloid equivalent value is calculated as follows:

$$\text{Colloid equivalent value (meq/g)} = 1/2 \times (\text{titrant volume for sample - titrant volume in blank test}) \times (\text{factor of N/400 PVSK})$$

The water-soluble vinyl polymer (A) of the present invention generally has a molecular weight corresponding to an intrinsic viscosity (measured in 1 N $NaNO_3$ at 30°C; unit: dl/g; hereinafter the same shall apply) of not less than 2. When it is less than 2, the molecular weight is so small that the polymer can hardly have the required characteristics. Since, generally, a higher molecular weight leads to higher flocculant performance, the intrinsic viscosity is preferably not less than 4, more preferably not less than 5, still more preferably not less than 8.

The above water-soluble vinyl polymer (A) can be obtained from the ketimine-containing (meth)acrylate (n) mentioned above under [3], by the production processes mentioned below.

The water-soluble vinyl polymer (A) can be produced by hydrolyzing and neutralizing, with an acidic aqueous solution, a polymer or copolymer of the ketimine-containing (meth)acrylate (n) represented by the above general formula (2). The water-soluble vinyl polymer (A) can also be produced by another process which comprises hydrolyzing and neutralizing the ketimine-containing (meth)acrylate (n) with an acidic aqueous solution, followed by polymerizing or

copolymerizing the resulting monomer.

Among these, the process comprising hydrolyzing and neutralizing the ketimine-containing (meth)acrylate (n) with an acidic aqueous solution, and polymerizing or copolymerizing the resulting monomer is simple, hence preferred.

The ketimine-containing (meth)acrylate (n), which is the starting material in the process for producing the above water-soluble vinyl polymer (A), can be prepared by the novel production method mentioned above under [4]. Thus, the ketimine-containing (meth)acrylate (n) represented by the above general formula (2) can be produced by transesterification of an alkyl (meth)acrylate with the ketimine compound of a primary amino alcohol as represented by the above general formula (4).

For producing the above monomer (m), which is the essential constituent monomer of the above water-soluble vinyl polymer (A), for example when the above monomer (m) is a monomer (m11) represented by the above general formula (3), there may be supposed such a method by transesterification of an alkyl (meth)acrylate with a primary amino alcohol. This method, however, the transesterification reaction is accompanied by such side reactions as amidation and Michael addition, hence the desired product cannot be obtained in a satisfactory yield.

On the contrary, in the processes of the present invention which comprises hydrolyzing and neutralizing a polymer or copolymer of the above ketimine-containing (meth)acrylate (n), or using, as a raw material for polymerization, the product of hydrolyzing and neutralizing the ketimine-containing (meth)acrylate (n), the procedure comprising hydrolyzing and neutralizing the blocked primary amino group resulting from ketiminization with an acidic aqueous solution, gives good yields and the side reaction problem during the transesterification reaction can be overcome. The processes are therefore very advantageous.

The above primary amino alcohol-derived ketimine of general formula (4) can be produced by a conventional method. For example, a primary amino alcohol and a ketone are mixed together, followed by dehydration with a dehydrating agent, such as sodium sulfate, potassium carbonate or molecular sieves, or by dehydration using an azeotropic solvent, for example an aliphatic hydrocarbon, such as cyclohexane or normal-hexane, an aromatic hydrocarbon, such as toluene; or ethyl acetate. Among these techniques, the azeotropic dehydration technique is industrially preferred, since the solvent can easily be recovered for reuse and no step is required for removing the dehydrating agent by filtration. Preferred as the azeotropic solvent are aliphatic hydrocarbons readily separable from water, in particular cyclohexane and normal-hexane.

As the, primary amino alcohol for ketiminization, there may be mentioned primary amino alcohols containing 2 to 4 carbon atoms, such as ethanolamine, propanolamine and 2,3-dihydroxypropylamine. When such an amino alcohol is used, Q in the general formula (2) representing the ketimine-containing (meth)acrylate (n) becomes an alkylene or hydroxyalkylene group containing 2 to 4 carbon atoms. Ethanolamine is preferred among these. The use of ethanolamine corresponds to the case in which Q is ethylene in the general formula (2) representing the ketimine-containing (meth)acrylate (n).

The ketone to be used for ketimine formation includes ketones having alkyl groups containing 1 to 4 carbon atoms, such as acetone, methyl ethyl ketone and methyl isobutyl ketone. Among the ketones, acetone and methyl ethyl ketone are preferred and methyl ethyl ketone is more preferred, because of ease of recovery. The use of methyl ethyl ketone corresponds to the case where, in the general formula (2) representing the ketimine-containing (meth)acrylate (n), $R^2$ is ethyl group and $R^3$ is methyl group.

The ketone is used preferably in an amount of 1.0 to 5 moles, more preferably 1.1 to 2.0 moles, per mole of the primary amine. It is preferred that the molar ratio is not more than 5, since a large-size reaction vessel is not necessary and a large amount of energy is not required for recovery and reuse of the ketone. On the other hand, a molar ratio not less than 1.0 does not cause any substantial decrease in reaction rate, hence is preferred.

Usable as the alkyl (meth)acrylate to be transesterified with the above ketimine are (meth)acrylates having an alkyl group containing 1 to 4 carbon atoms, for example methyl (meth)acrylate, ethyl (meth)acrylate and butyl (meth)acrylate. Preferred are methyl (meth)acrylates, which are easily removed.

The above (meth)acrylate is used preferably in an amount of 1.0 to 10 moles, more preferably 1.2 to 2.5 moles, per mole of the ketimine. A molar ratio not more than 10 is preferred since a large-size reaction vessel is not necessary and a large amount of energy is not required for the recovery and reuse of the unreacted portion of the alkyl (meth)acrylate.

The transesterification of the alkyl (meth)acrylate with the primary amino alcohol-derived ketimine compound of general formula (4) can be carried out in a conventional manner. Thus, the alkyl (meth)acrylate can be reacted with the ketimine compound of general formula (4), while removing the byproduct alcohol out of the system, with adding a catalyst thereto, in the presence of a polymerization inhibitor.

Catalysts for the above transesterification reaction include, for example, acidic catalysts such as sulfuric acid, p-toluenesulfonic acid and methanesulfonic acid; alkaline catalysts, such as sodium methoxide and potassium tert-butoxide; metal catalysts such as dibutyltin oxide, nickelacetylacetone complex, and the like. Preferred are metal catalysts.

As regards the reaction temperature and pressure for the above transesterification reaction, it is preferred for keeping a balance between main and side reactions to carry out the reaction under the reaction under normal pressure at 60°C to 130°C, particularly under normal pressure at 80°C to 120°C.

Usable as the above polymerization inhibitor are ones conventionally used, for example one or more of phenothiazine, cupferron, hydroquinone, p-monomethyl hydroquinone and the like.

The above polymerization inhibitor is used preferably in an amount of 0.01 to 1.0% by weight, more preferably 0.1 to 0.5% by weight, based on the weight of the alkyl (meth)acrylate.

It is preferred to purify through distillation the above transesterified product containing the polymerization inhibitor and the catalyst, which may affect adversely polymerizability of the monomer(s). Therefore, the product is preferably purified by distillation. For preventing polymerization within a fractionating tower on that occasion, a polymerization inhibitor solution may be showered in the tower.

Methods of obtaining a polymer or copolymer of the above ketimine-containing (meth)acrylate (n) include those by homopolymerizing the ketimine-containing (meth)acrylate (n) mentioned above under [4], and those by copolymerizing the same with another vinyl monomer (k). Examples of the other vinyl monomer (k) are the same ones as mentioned above.

The proportion of the monomer (n) in case copolymerized with the other vinyl monomer (k) may be selected arbitrarily according to the intended purpose. In view of performances of the polymer, particularly dehydration degree when used, as a high-molecular weight flocculant, of the product after hydrolysis and neutralization with an acidic aqueous solution by the procedure mentioned later herein, the proportion of the monomer (n) is preferably not less than 50 mole %, more preferably not less than 70 mole %, particularly not less than 80 mole %, based on the total of the cationic vinyl monomers.

For the same reasons, the amount of the monomer (n) based on the total of all the vinyl monomers, including the nonionic and anionic ones, is preferably not less than 50 mole %, more preferably not less than 70 mole %, still more preferably not less than 80 mole %.

The homopolymerization of the above ketimine-containing (meth)acrylate (n) or copolymerization thereof with another vinyl monomer (k) can be carried out in the conventional manner, for example through radical polymerization. Suitable radical polymerization techniques include, for instance, solution polymerization, emulsion polymerization using water and an organic solvent, and suspension polymerization. Solution polymerization is preferred.

Solvents usable in solution polymerization include, for example, isopropyl alcohol, acetone, methyl ethyl ketone, toluene, xylene, tetrahydrofuran, dimethylformamide, ethyl acetate and the like.

When such a solvent is used, the monomer concentration is preferably 10 to 80% by weight, more preferably 15 to 50% by weight.

In carrying out the polymerization, there may be used a common polymerization initiator (c) (for example, organic peroxide such as benzoyl peroxide; or azo compound such as 2,2'-azobis-isobutyronitrile or 2,2'-azobis-2-methylbutyronitrile).

In charging the raw materials, the ketimine-containing (meth)acrylate (n), other vinyl monomer (k) and polymerization initiator (c) may be charged all at once, or a solution of the ketimine-containing (meth)acrylate (n) and/or other vinyl monomer (k) and/or polymerization initiator (c) in a solvent as mentioned above may be added dropwise. Generally, a part or the whole of a solvent is charged in advance. The reaction temperature is preferably within the range of 60 to 140°C, more particularly 70 to 120°C.

The resulting polymer or copolymer of the ketimine-containing (meth)acrylate (n) by the above polymerization can be further hydrolyzed and neutralized by further mixing an acidic aqueous solution therewith into the corresponding polymer or copolymer having the primary amine salt group-containing (meth)acrylate units.

Species of the above acid include, for example, hydrochloric acid, hydrobromic acid, hydroiodic acid, nitric acid, sulfuric acid, methanesulfonic acid, phosphoric acid, and acetic acid. Among these, hydrochloric acid is preferred.

The above hydrolysis and neutralization are carried out preferably at a temperature of 0 to 60°C, especially 5 to 30°C considering the balance between the main and side reactions.

In the above hydrolysis and neutralization, the acidic aqueous solution is used in such an amount to adjust the pH after hydrolysis and neutralization in the range of 3.0 to 5.0, more preferably 3.4 to 4.6. Adjusting the pH to 3.0 to 5.0 is preferred for stability, without causing side reactions such as hydrolysis and Michael addition.

In cases of using the hydrolyzed and neutralized product from the above ketimine-containing (meth)acrylate, this hydrolyzed and neutralized product can be prepared by the production method mentioned hereinabove under [5]. Thus, the primary amine salt group-containing (meth)acrylate ester (m11) represented by the general formula (3) can be produced by subjecting the ketimine-containing (meth)acrylate (n) obtained by the production method mentioned above under [4] further to hydrolysis and neutralization with an acidic aqueous solution.

Acid species, reaction temperature and amount of acidic aqueous solution in the above hydrolysis and neutralization may be the same as those mentioned above referring to the hydrolysis and neutralization of the above polymer or copolymer of the ketimine-containing (meth)acrylate (n).

After hydrolysis and neutralization, the dissociated ketone is removed. Removing methods include, for example, liquid-liquid phase separation, removal by distillation under reduced pressure and so forth. Among these, the phase separation is preferred because of low energy consumption. In carrying out the phase separation, it is preferable to add a solvent such as normal-hexane to increase separability. In reusing the recovered ketone for ketimine formation, the mixture of the ketone and normal-hexane or the like recovered by phase separation can be used as such.

The primary amine salt group-containing (meth)acrylate ester (m11), which is the hydrolyzed and neutralized product of the above ketimine-containing (meth)acrylate (n), can be employed to produce the water-soluble vinyl polymer (A) of the present invention, by homopolymerizing or copolymerizing it optionally with said other vinyl monomer (k) as mentioned above, by routine techniques such as aqueous solution polymerization, emulsion polymerization using water and an organic solvent, and suspension polymerization.

Preferred as the method of polymerization is aqueous solution polymerization. In the case of aqueous solution polymerization, an aqueous monomer solution, preferably having a concentration of 10 to 80% by weight, is prepared. After substituting the atmosphere with an inert gas is added, a conventional polymerization catalyst [a persulfate salt such as ammonium persulfate or potassium persulfate; an organic peroxide such as benzoyl peroxide; an azo compound such as 2,2'-azobis(amidinopropane) hydrochloride or azobis(cyanovaleric acid); or a redox catalyst [a combination of a peroxide (e.g. $H_2O_2$, ammonium persulfate or potassium persulfate) and a reducing agent (e.g. sodium bisulfite or ferrous sulfate)]]. The polymerization is carried out, for instance, at about 20 to 100°C for several hours. It is also possible to add a photosensitizer and then irradiate the mixture with ultraviolet light or the like. For pulverization, the polymer thus obtained may be adequately cut into pieces and dried by exposure to hot air, or precipitated from a solvent and dried, and then ground.

The water-soluble vinyl polymer (A) of the present invention, as a product, may have an arbitrary conventional form, such as a powder, film, aqueous solution, w/o type emulsion or suspension.

The water-soluble vinyl polymer (A) of the present invention can judiciously be used as a high-molecular weight flocculant, particularly as a sludge dehydrating agent.

The high-molecular weight flocculant of the present invention, comprising the water-soluble vinyl polymer (A), shows high adsorbability of particles suspended in water and thus shows effective flocculating properties (floc-forming ability) and, therefore, can be used for promoting the solid-liquid separation of ordinary aqueous suspensions. From the practical value viewpoint, it is useful in dehydrating organic sludges (the so-called raw sludge, excess sludge, mixed raw sludge, digested sludge, coagulating sedimentation/pressure floatation sludge, and mixtures thereof) formed upon microbiological treatment of sewage, excreta and the like. For the same reasons, it is more useful in dehydrating sludges having an organic matter content (VSS/SS) of not less than 70% by weight.

The high-molecular weight flocculant of the present invention, comprising the water-soluble vinyl polymer (A), may be used singly or, according to the intended use, in combination with another high-molecular weight flocculant, such as one comprising a water-soluble cationic or amphoteric vinyl polymer (B) derived from a radical-polymerizable cationic vinyl monomer (m') as its essential monomer.

Exemplary of the above monomer (m') are (meth)acryloyloxyethyltrimethylammonium chloride, (meth)acryloyloxyethyldimethylbenzylammonium chloride, (meth)acryloylaminoethyltrimethylammonium chloride, dimethyldiallylammonium methyl sulfate, vinylpyridine and the like.

Illustrative examples of the above water-soluble vinyl polymer (B) include water-soluble cationic vinyl polymers such as polymethacryloyloxyethyltrimethylammonium chloride, acrylamide-acryloyloxyethyltrimethylammonium chloride copolymer and polyvinylamidine; and water-soluble amphoteric vinyl polymers such as acrylamide-acrylic acid-acryloyloxyethyltrimethylammonium chloride copolymer. Among them, preferred are water-soluble amphoteric vinyl polymers, and water-soluble cationic vinyl polymers having a colloid equivalent value at pH 4 of not less than 4.0, particularly polymethacryloyloxyethyltrimethylammonium chloride.

For dehydrating flocky sludge formed upon addition of the high-molecular weight flocculant comprising the water-soluble vinyl polymer (A) of the present invention, ordinary methods, for instance, centrifugal dehydration, beltpress dehydration, filter press dehydration, capillary dehydration, and the like can be employed. From the efficiency viewpoint, centrifugal dehydration and beltpress dehydration are preferred.

A 0.1% (by weight) aqueous solution of the high-molecular weight flocculant of the present invention, which comprises the water-soluble vinyl polymer (A), preferably has a pH of not more than 4.0, particularly not more than 3.0. When the aqueous solution has a pH of not more than 4.0, particularly good dehydration performance can be obtained.

For making the pH of the 0.1% (by weight) aqueous solution not more than 4.0, the high-molecular weight flocculant of the present invention comprising the water-soluble vinyl polymer (A) may be used together with an acidic substance. As the acidic substance, there can be used ordinary inorganic or organic acidic substance. Illustrative are mineral acids such as sulfuric acid, hydrochloric acid and phosphoric acid; inorganic acidic solid substances such as acid sodium phosphate, acid salt cake, ammonium chloride, ammonium sulfate, ammonium bisulfate and sulfamic acid; and organic acids such as oxalic acid. Among them, preferred are inorganic acidic solid substances, particularly sulfamic acid and acid salt cake, from the effect and economy viewpoint.

The high-molecular weight flocculant of the present invention, comprising the water-soluble vinyl polymer (A), is used preferably in the form of an aqueous solution having a concentration of about 0.1% by weight, when a thickening agent, loading agent, dispersant, preservative and/or inorganic salt may be used in addition to the above acidic substance, according to the intended purpose of use of the flocculant.

BEST MODE FOR CARRYING OUT THE INVENTION

The present invention is further explained by examples and comparative examples below, but the invention is not restricted to them.

Example 1

Into a three-necked flask equipped with a stirrer, a thermometer, a water-measuring tube and a condenser were charged 61 g (1.0 mole) of monoethanolamine, 86 g (1.2 moles) of methyl ethyl ketone and 100 g of normal-hexane, and reacted under reflux at 65°C to 80°C for 12 hours to remove water from the reaction system via the water-measuring tube. When the conversion reached not less than 98%, the reaction was stopped and the excess methyl ethyl ketone and normal-hexane were removed. The yield was 109 g (95%).

The water-measuring tube was replaced with a fractionating column, 190 g (1.9 moles) of methyl methacrylate, 0.95 g of phenothiazine and 0.57 g of zinc acetylacetonate complex were added, and reacted for 10 hours maintaining the column head temperature at 60°C to 65°C (system inside temperature 95 to 110°C) and azeotropic mixture of methanol and methyl methacrylate was removed from the system. The conversion was 80%. Then, the excess methyl methacrylate was distilled off in the conventional manner under reduced pressure (100 mm Hg) and the main product was further purified by distillation under reduced pressure (10 mm Hg). The yield of the main product of a ketimine-containing methacrylate was 125 g (90% based on the ketimine consumed).

Another flask was charged with 100 g of the main product, which was neutralized, maintaining the reaction temperature at 25°C, to pH 4 by gradual addition of an aqueous hydrochloric acid solution. Thereafter, normal-hexane was added, followed by stirring the resulting mixture for an hour, and then taking out the lower aqueous layer to obtain an aqueous solution of aminoethyl methacrylate hydrochloride. The yield of the aqueous solution was 115 g (solid content 86.5%, yield 98%).

This product was adjusted to a concentration of 50% by weight using deionized water. After substituting the atmosphere with nitrogen and adjusting the temperature to 35°C, ammonium persulfate and sodium bisulfite were added in that order each in an amount of 0.015% by weight based on the monomer, which was subjected to polymerization for 5 hours. The polymerization product (gel) obtained was dried with hot air at 100°C and then ground to give a powdery homopolymer of aminoethyl methacrylate hydrochloride.

Comparative Example 1

A mixture of 61 g (1.0 mole) of monoethanolamine, 200 g (2.0 moles) of methyl methacrylate, 0.1 g of phenothiazine and 0.6 g of zinc acetylacetonate complex was heated at 95 to 110°C to effect transesterification. The desired transesterification product was not obtained. The main products were amidation and Michael addition products.

Example 2

A flask equipped with a stirrer, a thermometer, two dropping funnels and a condenser was charged with 200 g of toluene, and one dropping funnel [1] was charged with a solution of 183 g (1.0 mole) of the ketimine-containing methacrylate obtained as an intermediate in Example 1 in 100 g of toluene and the other dropping funnel [2] with a solution of 0.5 g of 2,2'-azobis-isobutyronitrile in 20 g of toluene. After raising the system inside temperature to 80°C, dropping was started from both the funnels [1] and [2] simultaneously. While the reaction temperature was maintained at 80°C, the dropping was conducted and completed in 2 hours. After completion of the dropping, the mixture was matured at 100°C for 5 hours to give a homopolymer of the ketimine-containing methacrylate. The yield was 500 g (99%, polymer concentration 36%).

After cooling to 25°C and while maintaining the reaction temperature at 25°C, an aqueous hydrochloric acid solution was gradually added for neutralization to pH 4, followed by separating the aqueous phase from the toluene phase to give an aqueous solution of a homopolymer of aminoethyl methacrylate hydrochloride.

Examples 3 to 6 and Comparative Examples 2 to 4

The monomer specified in Table 1 was subjected to polymerization in the same manner as in Example 1.

Each high-molecular weight flocculant thus obtained was dissolved in deionized water and measured for colloid equivalent values at pH of 4, 7 and 10.

Table 1

| | | High-molecular weight flocculant | Colloid equivalent value (meq/g) | | | | |
|---|---|---|---|---|---|---|---|
| | | Monomer composition (molar ratio) | pH4 a | pH7 b | pH10 c | b/a | c/a |
| Example | 3 | AEMC(100) | 6.0 | 2.4 | 0.1 | 0.40 | 0.017 |
| | 4 | AEAS(100) | 6.6 | 2.7 | 0.1 | 0.41 | 0.015 |
| | 5 | AEMC(80)-AAm(20) | 5.5 | 2.2 | 0.1 | 0.40 | 0.018 |
| | 6 | AEMC(50)-AAm(50) | 4.2 | 1.6 | 0.1 | 0.38 | 0.024 |
| Compar. Example | 2 | METAC(100) | 4.8 | 4.6 | 4.5 | 0.96 | 0.94 |
| | 3 | METAS(100) | 3.6 | 3.5 | 3.4 | 0.97 | 0.94 |
| | 4 | METAC(50)-AAm(50) | 3.6 | 2.2 | 1.2 | 0.61 | 0.33 |
| AEMC: Aminoethyl methacrylate hydrochloride<br>AEAS: Aminoethyl acrylate sulfate<br>METAC: Methacryloyloxyethyltrimethylammonium chloride<br>METAS: Methacryloyloxyethyltrimethylammonium methyl sulfate<br>AAm: Acrylamide | | | | | | | |

Examples 7 to 10 and Comparative Example 5 to 7

The monomer specified in Table 2 was subjected to polymerization in the same manner as in Example 1. Each high-molecular weight flocculant thus obtained was dissolved in deionized water to give a 0.1% aqueous solution. This was added to excess sludge from a sewage treatment plant in K city [pH 6.9; TS (total solids) 2.3%; organic matter 81%] in an amount of 0.85%/TS, followed by carrying out dehydration with a small-size beltpress dehydrator and measuring water content of the resulting dehydrated cake.

The test results are shown in Table 2. The high-molecular weight flocculants of the present invention (Examples 7 to 10) showed higher dehydration efficiency (low cake water contents) as compared with Comparative Examples 5 to 7.

Table 2

| | | High-molecular weight flocculant | | Sludge dewatering test results |
|---|---|---|---|---|
| | | Monomer composition (molar ratio) | Intrinsic viscosity (dl/g) | Cake water content (%) |
| Example | 7 | AEMC(100) | 5.8 | 77.5 |
| | 8 | AEAS(100) | 5.6 | 77.8 |
| | 9 | AEMC(80)-AAm(20) | 6.2 | 78.0 |
| | 10 | AEMC(50)-AAm(50) | 6.8 | 78.6 |

Table 2 (continued)

| | | High-molecular weight flocculant | | Sludge dewatering test results |
|---|---|---|---|---|
| | | Monomer composition (molar ratio) | Intrinsic viscosity (dl/g) | Cake water content (%) |
| Compar. Example | 5 | METAC(100) | 5.9 | 81.8 |
| | 6 | METAS(100) | 5.8 | 82.2 |
| | 7 | METAC(50)-AAm(50) | 6.9 | 83.6 |
| Notes) The symbols are as defined under Table 1. | | | | |

INDUSTRIAL APPLICABILITY

The present invention provides a novel water-soluble, primary amine salt group-containing vinyl polymer, a process for producing the same and processes for producing a precursor and intermediate for the polymer.

The methods of the invention make it possible to produce precursors and intermediates, such as a ketimine-containing (meth)acrylate, a primary amine salt group-containing (meth)acrylate, a polymer or copolymer of the ketimine-containing (meth)acrylate, in good yields on a commercial scale.

The polymer of the invention is useful as a high-molecular weight flocculant, a paper strength-improving agent, a paper drainage aid and an antistatic agent and has good dehydrating effects particularly on organic sludges resulting from microbiological treatment of sewage and excreta. The high-molecular weight flocculant is particularly useful as a sludge dehydrating agent, which is capable of attaining remarkable effects on sludges of a high organic matter content (VSS/SS), having so far been difficult to dewater.

**Claims**

**1.** A water-soluble vinyl polymer (A) consisting essentially of constituent monomer units of a radical-polymerizable (meth)acrylic monomer (m), containing a primary amine salt group,

said polymer (A) having colloid equivalent values a, b and c, at pH of 4, 7 and 10, respectively, providing the ratio of b/a in the range of 0.1 - 0.5 and the ratio of c/a in the range of 0 to 0.1.

**2.** The water-soluble vinyl polymer (A) according to Claim 1,

wherein the radical-polymerizable (meth)acrylic monomer (m) is a monomer (m1) represented by the general formula (1):

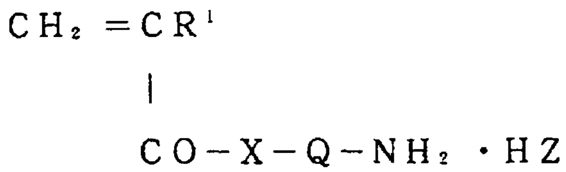

(1)

wherein $R^1$ is a hydrogen atom or a methyl group, X is an oxygen atom or an imino group, preferably an oxygen atom, Q is an alkylene or hydroxyalkylene group containing 2 to 4 carbon atoms, preferably an ethylene group, and Z is selected from the group consisting of Cl, Br, I, $NO_3$, $1/2SO_4$, $CH_3SO_3$, $H_2PO_4$ and $CH_3COO$.

**3.** The water-soluble vinyl polymer (A) according to Claim 2,

wherein said monomer (m1) is a (meth)acrylate ester (m11), containing a primary amine salt group and represented by the general formula (3):

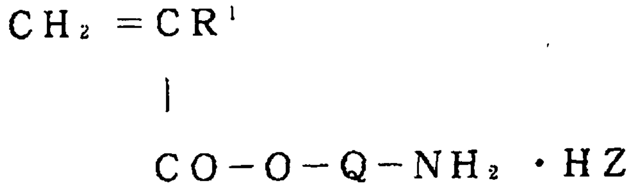

(3)

wherein $R^1$ is a hydrogen atom or a methyl group, Q is an alkylene or hydroxyalkylene group containing 2 to 4 carbon atoms, preferably an ethylene group, and Z is selected from the group consisting of Cl, Br, I, $NO_3$, $1/2SO_4$, $CH_3SO_3$, $H_2PO_4$ and $CH_3COO$,

said (meth)acrylate ester (m11) being obtainable by hydrolyzing and neutralizing, with an acidic aqueous solution, a ketimine-containing (meth)acrylate (n) represented by the general formula (2):

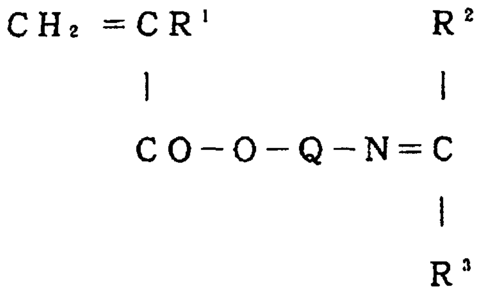

(2)

wherein $R^1$ and Q are the same as above, and $R^2$ and $R^3$ are alkyl groups containing 1 to 4 carbon atoms, preferably $R^2$ is an ethyl group and $R^3$ is a methyl group.

**4.** The water-soluble vinyl polymer (A) according to Claim 1, 2 or 3

which is a copolymer further comprising constituent monomer units of acrylamide and/or another cationic vinyl monomer and containing said radical-polymerizable (meth) acrylic monomer (m) in an amount of at least 50 % based on the total moles of the cationic vinyl monomers in the copolymer.

**5.** The water-soluble vinyl polymer (A) according to Claim 1, 2, 3 or 4

which has an intrinsic viscosity of at least 4 dl/g as measured in 1 N $NaNO_3$ at 30°C.

**6.** Use of the water-soluble vinyl polymer (A) according to Claim 1, 2, 3, 4, or 5,

wherein said water-soluble vinyl polymer (A) has an intrinsic viscosity of at least 4 dl/g as measured in 1N $NaNO_3$ at 30°C.

**7.** A process for producing the water-soluble vinyl polymer (A) according to Claim 3, 4 or 5,

comprising hydrolyzing and neutralizing, with an acidic aqueous solution, a polymer or copolymer comprising units of a ketimine-containing (meth)acrylate (n) represented by the general formula (2):

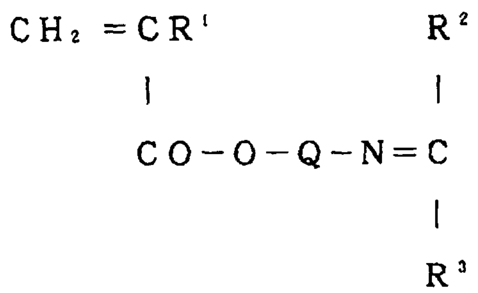

(2)

wherein $R^1$ is a hydrogen atom or a methyl group, Q is an alkylene or hydroxyalkylene group containing 2 to 4 carbon atoms, preferably an ethylene group, and $R^2$ and $R^3$ are alkyl groups containing 1 to 4 carbon atoms, preferably $R^2$ is an ethyl group and $R^3$ is a methyl group.

**8.** A process for producing the water-soluble vinyl polymer (A) according to Claim 3, 4 or 5,

comprising polymerizing or copolymerizing a product obtainable by hydrolyzing and neutralizing, with an acidic aqueous solution, a ketimine-containing (meth)acrylate (n) represented by the general formula (2):

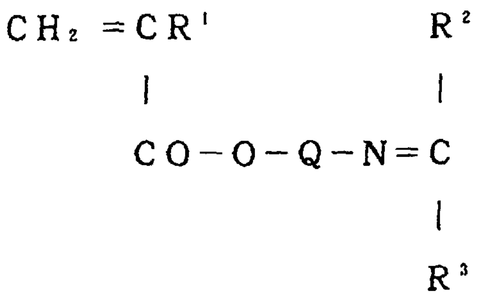

(2)

wherein $R^1$ is a hydrogen atom or a methyl group, Q is an alkylene or hydroxyalkylene group containing 2 to 4 carbon atoms, preferably an ethylene group, and $R^2$ and $R^3$ are alkyl groups containing 1 to 4 carbon atoms, preferably $R^2$ is an ethyl group and $R^3$ is a methyl group.

**9.** A process for producing a ketimine-containing (meth)acrylate (n) represented by the general formula (2):

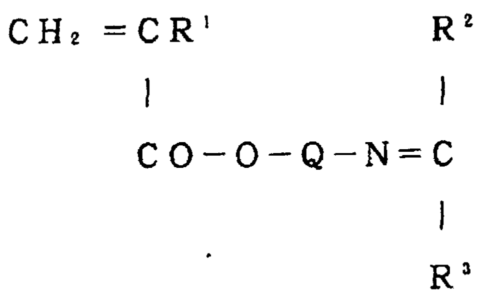

(2)

wherein $R^1$ is a hydrogen atom or a methyl group, Q is an alkylene or hydroxyalkylene group containing 2 to 4 carbon atoms, preferably an ethylene group, and $R^2$ and $R^3$ are alkyl groups containing 1 to 4 carbon atoms, preferably $R^2$ is an ethyl group and $R^3$ is a methyl group;

which process comprises carrying out transesterifying, with an alkyl (meth)acrylate a primary amino alcohol-derived ketimine represented by the general formula (4):

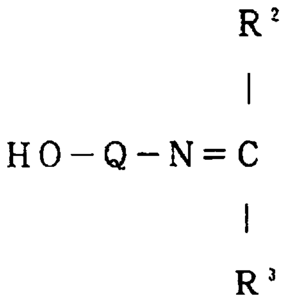

(4)

wherein Q is an alkylene or hydroxyalkylene group containing 2 to 4 carbon atoms, preferably an ethylene group, and $R^2$ and $R^3$ are alkyl groups containing 1 to 4 carbon atoms, preferably $R^2$ is an ethyl group and $R^3$ is a methyl group.

**10.** A process for producing a primary amine salt group-containing (meth)acrylate ester (m11) represented by the general formula (3):

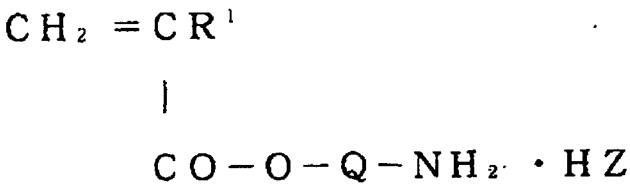

(3)

wherein $R^1$ is a hydrogen atom or a methyl group, Q is an alkylene or hydroxyalkylene group containing 2 to 4 carbon atoms, preferably an ethylene group, and Z is selected from the group consisting of Cl, Br, I, $NO_3$, $1/2SO_4$, $CH_3SO_3$, $H_2PO_4$ and $CH_3COO$;

which process comprises further hydrolyzing and neutralizing, with an acidic aqueous solution, the ketimine-containing (meth)acrylate obtainable by the process for producing according to Claim 9.

**11.** A process for producing a polymer or copolymer comprising units of a ketimine-containing (meth)acrylate

which process comprises polymerizing the ketimine-containing (meth)acrylate (n) obtainable by the process for producing according to Claim 9 or
copolymerizing said ketimine-containing (meth)acrylate (n) with another vinyl monomer (k).

## INTERNATIONAL SEARCH REPORT

International application No.
PCT/JP97/03824

**A. CLASSIFICATION OF SUBJECT MATTER**

Int. Cl[6] C08F20/34, 20/60, 8/12, C07C251/08, 219/08, 249/02, 213/02, C02F11/14, B01D21/01

According to International Patent Classification (IPC) or to both national classification and IPC

**B. FIELDS SEARCHED**

Minimum documentation searched (classification system followed by classification symbols)

Int. Cl[6] C08F20/34, 20/60, 8/12, 220/34, 220/60, 120/34, 120/06, C07C251/08, 219/08, 249/02, 213/02, C02F11/14, B01D21/01

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP, 58-139799, A (Kurita Water Industries Ltd.), August 19, 1983 (19. 08. 83), Claims 1 to 5; page 2, lower left column, line 18 to page 3, upper right column, line 1; Page 4, Table 1 (Family: none) | 1, 2, 4, 5, 6 |
| X | JP, 61-200898, A (Kurita Water Industries Ltd.), September 5, 1986 (05. 09. 86), Claims 1 to 3; page 2, lower right column, line 8 to page 3, upper right column, line 8 (Family: none) | 1, 2, 4, 5, 6 |
| A | JP, 7-171600, A (Kurita Water Industries Ltd.), July 11, 1995 (11. 07. 95), Claims 1, 2; Par. Nos. (0009), (0010); page 4, Table 1 (Family: none) | 1 - 6 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| February 10, 1998 (10. 02. 98) | February 24, 1998 (24. 02. 98) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP97/03824

C (Continuation). DOCUMENTS CONSIDERED TO BE RELEVANT

| Category* | Citation of document, with indication, where appropriate, of the relevant passages | Relevant to claim No. |
|---|---|---|
| A | JP, 4-59100, A (Japan Sewage Works Agency, Kurita Water Industries Ltd.), February 25, 1992 (25. 02. 92), Claim 1; page 3, upper left column, line 5 to lower right column, 3rd line from the bottom; page 6, Tables 1, 2 (Family: none) | 1 - 6 |
| X | JP, 38-8310, B1 (Rohm and Haas Co.), June 7, 1963 (07. 06. 63), Page 1, left column, 15th line from the bottom to right column, 14th line from the bottom; page 2, left column, 11th line from the bottom to page 3, left column, line 8; page 4, right column, line 29 to page 6, left column, Examples 1 to 4 (Family: none) | 9 - 11<br>1 - 8 |

Form PCT/ISA/210 (continuation of second sheet) (July 1992)

INTERNATIONAL SEARCH REPORT

International application No.
PCT/JP97/03824

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

Claims 9 to 11 pertain to processes for the production of monomers and polymers. Since, however, the processes of claims 9 to 11 do not directly relate to a water-soluble polymer of claim 1 specified in the colloidal equivalent, claims 9 to 11 relate merely to processes for production in themselves and thus are different from claims 1 to 8 in the problems, though the processes of claims 9 to 11 have a possibility of giving the polymer of claim 1. Accordingly, there is no matter common to all of the claims. Since there is no other common matter considered as being a special technical feature in the sense of the second sentence of Rule 13.2 of the Regulations under the PCT, there cannot be found among claims 1 to 11 any technical linkage in the sense of Rule 13 of the Regulations under the PCT.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

Remark on Protest
☐ The additional search fees were accompanied by the applicant's protest.
☐ No protest accompanied the payment of additional search fees.

Form PCT/ISA/210 (continuation of first sheet (1)) (July 1992)